# EUROPEAN PATENT APPLICATION

(11) **EP 2 974 790 A1**
(43) Date of publication of application: **20.01.2016**
(21) Application number: 15176509.6
(22) Date of filing: 13.07.2015
(51) Int. Cl.: B01J 20/289, B01J 20/32, B01D 15/38, C07K 1/22

(54) **ADSORBENT**

(30) Priority: 18.07.2014 JP 2014147291
(71) Applicant: Hitachi, Ltd., Tokyo 100-8280 (JP)
(72) Inventor: Maruyama, Masashi, Tokyo, 100-8280 (JP); Tada, Yasuhiko, Tokyo, 100-8280 (JP); Shibuya, Keisuke, Tokyo, 100-8280 (JP)
(74) Representative: Beetz & Partner mbB

(57) **Abstract**

An adsorbent of the present invention includes a support (20); and a protein(10) having an adsorption site (30)for adsorbing a target substance, in which the protein includes a reversible binding site (40) and a covalent binding site (50), the support includes an orientation-controlling site (70)forming a reversible bond with the reversible binding site(40), and an immobilization site(80) forming a covalent bond with the covalent binding site (50), the covalent binding site (50) includes a nucleophilic functional group, and the immobilization site (80)includes a functional group capable of reacting with the nucleophilic functional group by a nucleophilic substitution or a nucleophilic addition. Thus, it is possible to provide an adsorbent in which the utilization efficiency of the adsorption site of the protein is improved. As a result, the protein amount can be reduced, and the step for storing a culture solution can be simplified due to the faster purification step.

## Description

### Technical Field:

The present invention relates to an adsorbent on which a protein is immobilized and relates to an adsorbent used for example for purification of biopharmaceuticals.

### Background Art:

A technique for immobilizing a functional biomolecule on a support and using the biomolecule is used in a wide field including antibody purification, analysis devices, cell culture, recovery of microorganisms, enzyme electrodes, screening of medicines and the like.

Among them, a protein capable of adsorbing a target substance is widely used irrespective of field and is of importance. In this regard, adsorption sites of such a protein are often localized on a part of the protein, and the adsorption sites cannot always be used for steric reasons when the protein is immobilized on the support.

Techniques for immobilizing the protein on the support include a method using physical adsorption, a method for immobilizing via an amino group and the like. However, in both methods, it is difficult to control the orientation and a utilization efficiency of the adsorption sites of the protein is an issue to be addressed. Thus, development of a technique for expressing a certain sequence in a protein by a genetic recombination and controlling the orientation using the sequence has been desired.

As a technique for immobilizing a protein with the sequence expressed by the genetic recombination, it is reported that cysteine is added to the C-terminus of protein A and a thiol group of the cysteine is used as described in JP-A-2008-101023 (Patent Literature 1), but this method is not always effective.

As a technique for immobilizing a protein using a purification tag for controlling the orientation, a method for reacting a support with a carbodiimide is reported, the support containing a protein bound via a HAT-tag to its surface having a metal complex, as described in JP-T-2007-529487 (Patent Literature 2). However, the control of the orientation may be disturbed by the reaction between the metal complex and the carbodiimide, and the activity may be decreased or the protein may be immobilized with unfavorable orientation when the carboxyl groups in the protein react with the carbodiimide.

Moreover, a method for applying an ultraviolet light to a support is reported, the support containing a protein bound via a His tag to its surface having a metal complex and a photoreactive site, as described in Langmuir 2013, 29, 11687-11694 (Non-Patent Literature 1). However, it is difficult to apply this method to an adsorbent because the protein activity decreases by the immobilization and productivity of the photoreaction is low.

### Summary of Invention:

An adsorbent of the present invention includes a support; and a protein having an adsorption site for adsorbing a target substance, in which the protein includes a reversible binding site and a covalent binding site, the support includes an orientation-controlling site forming a reversible bond with the reversible binding site, and an immobilization site forming a covalent bond with the covalent binding site, the covalent binding site includes a nucleophilic functional group, and the immobilization site includes a functional group capable of reacting with the nucleophilic functional group by a nucleophilic substitution or a nucleophilic addition.

According to the present invention, it is possible to provide an adsorbent in which the utilization efficiency of the adsorption site of the protein is improved. As a result, the protein amount can be reduced, and the step for storing a culture solution can be simplified due to the faster purification step.

### Brief Description of the Drawings:

Fig. 1 is a schematic view showing an adsorbent of the present invention.
Fig. 2 is a flow chart showing a production method of the adsorbent of Fig. 1.
Fig. 3 is a flow chart showing a production method of a support.
Fig. 4 shows a flow chart of an antibody-preparation production process and a schematic view of a purification column.

### Description of Embodiments:

Although an adsorbent which has a structure of immobilizing a protein having an adsorption site on a support has been used for purification of biopharmaceuticals and analysis devices, the protein orientation has been at random and the utilization efficiency of the adsorption site has been low.

An object of the present invention is to provide an adsorbent which has the improved utilization efficiency of the adsorption site of the protein.

An adsorbent of the present invention is an adsorbent containing a protein having an adsorption site for adsorbing a target substance immobilized via a support and is characterized in that the support has a support base material, an orientation-controlling site forming a reversible bond with a reversible binding site of the protein and an immobilization site thermally reacting with a covalent binding site of the protein to form a covalent bond, and the formation process of the reversible bond progresses earlier than the formation process of the covalent bond.

Embodiments of the present invention are explained below using figures. The following explanations show embodiments of the present invention, and the present invention is not limited by the explanations. In addition, in all the figures, those which have the same function are indicated by the same symbol and a part of the explanation is sometimes omitted.

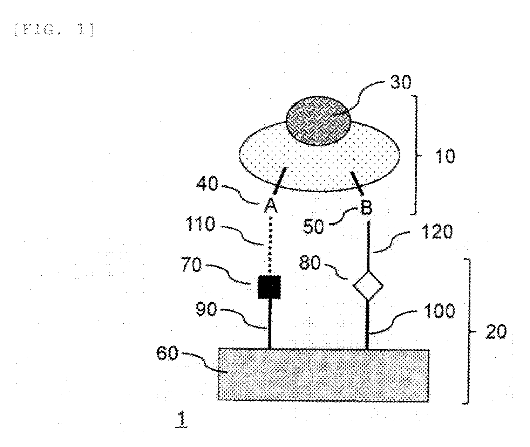

Fig. 1 shows an adsorbent obtained using the present invention.

In this figure, an adsorbent 1 has a protein 10 and a support 20.

The protein 10 has an adsorption site 30, a reversible binding site 40 and a covalent binding site 50.

The support 20 has a support base material 60, an orientation-controlling site 70 and an immobilization site 80 (a site for immobilization). Between the support 60 and the orientation-controlling site 70, an orientation-controlling site spacer 90 is interposed as a linking group bound chemically. Between the support 60 and the immobilization site 80, an immobilization site spacer 100 is interposed as a linking group bound chemically.

Between the reversible binding site 40 of the protein 10 and the orientation-controlling site 70 of the support 20, a reversible bond 110 may be interposed as a linking group bound chemically. The reversible bond 110 is a coordinate bond between a metal ion and a ligand. After formation of the reversible bond 110 and the covalent binding site 50, the reversible bond 110 may disappear during a washing process. Thus, the reversible binding site 40 and the orientation-controlling site 70 may be bonded through the reversible bond 110 and may be bonded without the reversible bond 110 after the formation of the reversible bond 110 and the covalent binding site 50.

The covalent binding site 50 of the protein 10 and the immobilization site 80 of the support 20 are connected by a covalent bond 120.

The protein 10 is a peptide or protein which is natural or genetically modified, and examples are protein A, protein G, protein L, an immunoglobulin, avidin, streptavidin, a lectin, a kinesin, a conjugated protein containing the proteins and a genetically modified protein thereof, although the protein 10 is not limited to the examples.

More than one adsorption sites 30 may be on the protein 20, or only one adsorption site 30 may be on the protein 20.

The reversible binding site 40 is a functional group capable of reversibly binding to the orientation-controlling site 70 of the support, and the reversible binding site 40 is preferably a peptide or a protein which can be introduced by a genetic recombination in view of the productivity. Examples are a His-tag, an HQ-tag, an HN-tag, a HAT-tag, glutathione, a glutathione-S-transferase and a maltose-binding protein, and a His-tag is more preferable, although the reversible binding site 40 is not limited to the examples. The His-tag is one of tag peptides consisting of about six sequential histidine residues (His residues). The HQ-tag is a peptide tag (HQHQHQ) forming by bonding a histidine and a glutamine with each other. The HN-tag is a peptide tag (HNHNHNHNHNHN) forming by bonding a histidine and an asparagine with each other. The HAT-tag is a peptide tag (KDHLIHNVHKEEHAHAHNK) derived from a lactic acid dehydrogenase of a fowl. The His-tag, HQ-tag, HN-tag and HAT-tag include at least two parts (two or more parts) of histidine. As a peptide tag including more parts of histidine, the reversible bond can be formed more easily. The His-tag including six parts of histidine can form the reversible bond particularly easily.

The covalent binding site 50 is a functional group capable of potentially reacting with the immobilization site 80 and forming a covalent bond. In view of the productivity, it is preferable to use a functional group which the protein generally has without requiring the genetic recombination as the covalent binding site, and the covalent binding site 50 is more preferably a nucleophilic functional group such as an alcohol, phenol, an amine, an imine, imidazole, a thiol, a sulfide, a disulfide, a carboxylic acid or a conjugate base thereof, and further preferably an amine, an imine, imidazole, a thiol or a conjugate base thereof, although it is not limited to the examples.

The support base material 60 is a plate, bead, fibrous, membrane or monolithic solid and preferably made of a material containing a polysaccharide, a synthetic resin, an inorganic compound or a composite material thereof. More preferably, the support base material 60 contains any of agarose, sepharose, cellulose, a polystyrene, a polyalkyl methacrylate, a polyglycidyl methacrylate, a polyvinyl alcohol, a polyvinylpyrrolidone, a polyacrylamide, a polysiloxane, a polyfluoroethylene, silica, alumina, titania, zirconia, iron oxide, ferrite, hydroxyapatite and silicate and further preferably contains any of agarose, sepharose, cellulose, a polystyrene, silica, iron oxide and ferrite, although it is not limited to these materials.

The orientation-controlling site 70 is a functional group which specifically binds to the reversible binding site 40, and examples are a metal complex having a ligand of denticity of three or greater, a peptide, a protein, a protein-mimicking molecule, sugar, a nucleic acid and an aptamer. A complex of nitrilotriacetic acid, iminodiacetic acid or 1,4,7-triazacyclononane with iron, cobalt, nickel, copper or zinc, glutathione, a glutathione-S-transferase and maltose are preferable, and the metal complex of nitrilotriacetic acid or iminodiacetic acid is more preferable in view of the productivity.

The immobilization site 80 is preferably a functional group capable of potentially reacting with a nucleophilic functional group by nucleophilic substitution or nucleophilic addition and has a structure containing for example ethylene oxide, propylene oxide, butylene oxide, cyclopentene oxide, cyclohexene oxide, cyclopropane, cyclooctyne, a carboxylate ester, a phosphate ester, a sulfate ester, a lactone, a lactam, a sultone, a tosylate, a mesylate or a triflate. A structure containing ethylene oxide is more preferable, but the immobilization site 80 is not limited to the examples. Ethylene oxide, propylene oxide, butylene oxide, cyclopentene oxide and cyclohexene oxide described above are epoxide sites used in Examples.

The orientation-controlling site spacer 90 is linear or branched, desirably of 0 to 100 Å, more desirably of 1 to 50 Å, further desirably of 5 to 20 Å and contains a polyethylene glycol, a polymethacrylic acid derivative or a polyacrylamide derivative for example.

The immobilization site spacer 100 is linear or branched, desirably of 0 to 100 Å, more desirably of 1 to 50 Å, further desirably of 5 to 20 Å and contains a polyethylene glycol, a polymethacrylic acid derivative or a polyacrylamide derivative for example.

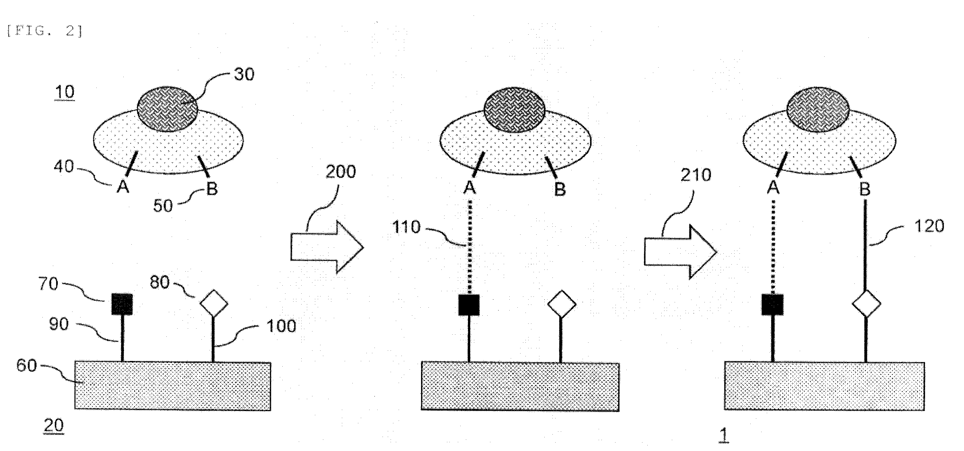

Fig. 2 shows a production method of the adsorbent 1. The adsorbent 1 can be produced by reacting the protein 10 and the support 20, and the production process contains a reversible bond formation process 200 and a covalent bond formation process 210.

When the reversible bond formation process 200 progresses earlier than the covalent bond formation process 210, the protein 10 is immobilized with the controlled orientation and the adsorption capacity of the adsorbent 1 increases as compared to the state in which the orientation is not controlled. The proportion of the proteins 10 immobilized while the reversible bond formation process 200 progresses earlier than the covalent bond formation process 210 in the total amount of the immobilized proteins 10 is preferably 10% or more, more preferably 40% or more and further preferably 80% or more. In this regard, because the reaction rate can easily change with the pH, steric hindrance, influence of coexisting ions and the like, the application of a combination of a known reversible bond formation process and a covalent bond formation process whose reaction is considered to be faster than that of the reversible bond formation process to the present invention is not necessarily excluded.

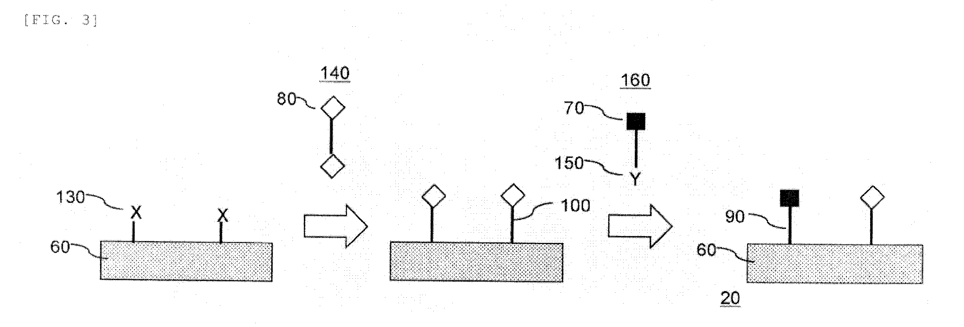

Fig. 3 is an example of a production method of the support 20. For example, the support 20 can be produced by a reaction between a reactive surface functional group 130 and a compound 140 having the immobilization sites 80 and a next reaction between the immobilization site 80 and an orientation-controlling site raw material 160 having the orientation-controlling site 70 and a reactive functional group 150.

The reactive surface functional group 130 is for example an alcohol, phenol, an amine, an imine, a thiol or a conjugate base thereof and the amine is desirable in view of the productivity.

The reactive functional group 150 is for example an alcohol, phenol, an amine, an imine, a thiol or a conjugate base thereof and the amine is desirable in view of the productivity.

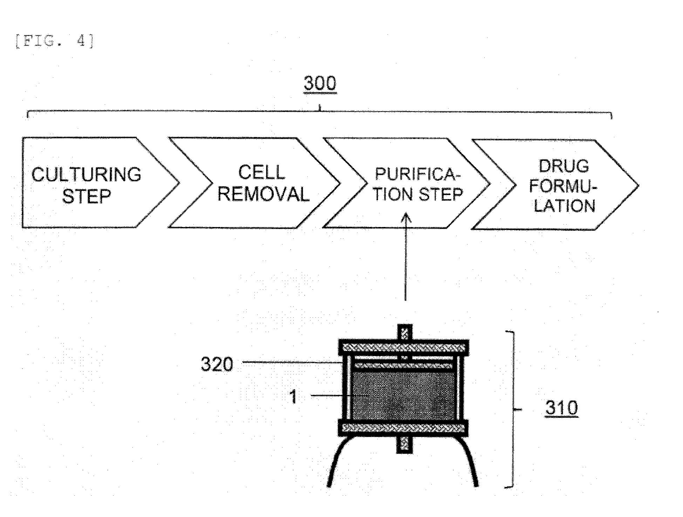

Fig. 4 shows an antibody-preparation production step 300 consisting of a culturing step, cell removal, a purification step and drug formulation and shows an example of the applications of the adsorbent 1. A purification column 310 is a purification device produced by filling an empty column 320 with the adsorbent 1, and the purification step can be carried out using the purification column 310.

### [Example 1]

In Example 1, a combination in which the rate of the covalent bond formation was low and the reversible bond formation process progressed earlier than the covalent bond formation process was investigated.

A support having amino groups on its surface was reacted with an aqueous solution containing 1,4-butanediol diglycidyl ether (10 wt%) and potassium sulfate (0.4 M) at 60°C for eight hours, and thus epoxide sites were introduced onto a surface of the support. Then, by reacting with a buffer solution (pH 10) of NαNα-carboxymethyllysine (5 wt%) at 60°C for two hours, nitrilotriacetic acid sites were introduced to about 40% of the epoxide sites. Then, by reacting the support obtained with a 0.1 M aqueous solution of nickel chloride, the nitrilotriacetic acid sites were converted into nickel complexes. By reacting the support thus obtained with a buffer solution (pH 7.4) of protein A (0.01 wt%) having a His-tag site at 37°C for eight hours, an adsorbent on which protein A was immobilized was obtained. Herein, a reversible binding site is the His-tag site, a covalent binding site is an amino group included in the protein A, an orientation-controlling site is the nitrilotriacetic acid sites, and the immobilization sites are the epoxide sites. Further, the unit "wt%" described above means "percent by weight".

### [Example 2]

In Example 2, a combination in which the rate of the covalent bond formation was low and the reversible bond formation process progressed earlier than the covalent bond formation process was investigated.

A support having amino groups on its surface was reacted with an aqueous solution containing polyethylene glycol diglycidyl ether (10 wt%) and potassium sulfate (0.4 M) at 60°C for eight hours, and thus epoxide sites were introduced onto a surface of the support. Then, by reacting with a buffer solution (pH 10) of NαNα-carboxymethyllysine (5 wt%) at 60°C for two hours, nitrilotriacetic acid sites were introduced to about 23% of the epoxide sites. Then, by reacting the support obtained with a 0.1 M aqueous solution of nickel chloride, the nitrilotriacetic acid sites were converted into nickel complexes. By reacting the support thus obtained with a buffer solution (pH 7.4) of protein A (0.01 wt%) having a His-tag site at 37°C for eight hours, an adsorbent on which protein A was immobilized was obtained.

### [Example 3]

In Example 3, a combination in which the rate of the covalent bond formation was low and the reversible bond formation process progressed earlier than the covalent bond formation process was investigated.

A support having amino groups on its surface was reacted with an aqueous solution containing polyethylene glycol diglycidyl ether (10 wt%) and potassium sulfate (0.4 M) at 60°C for eight hours, and thus epoxide sites were introduced onto a surface of the support. Then, by reacting with a buffer solution (pH 10) of NαNα-carboxymethyllysine (5 wt%) at 60°C for four hours, nitrilotriacetic acid sites were introduced to about 40% of the epoxide sites. Then, by reacting the support obtained with a 0.1 M aqueous solution of nickel chloride, the nitrilotriacetic acid sites were converted into nickel complexes. By reacting the support thus obtained with a buffer solution (pH 7.4) of protein A (0.01 wt%) having a His-tag site at 37°C for eight hours, an adsorbent on which protein A was immobilized was obtained.

### [Example 4]

In Example 4, an application of the adsorbent obtained by the present invention was investigated.

An empty column with a diameter of 60 cm was filled with the adsorbent obtained according to Example 3 to a height of 20 cm with a PBS buffer solution (pH 7.4). Herein, the PBS buffer solution is a phosphate buffer saline. A buffer solution (100 L) containing IgG (1000 g) and contaminants was added to the purification column thus produced at a flow rate of 200 cm/h, and the adsorbent was washed with the PBS buffer solution (pH 7.4) at a flow rate of 200 cm/h. Then, a citric acid buffer solution (pH 3.5) was added to the column at a flow rate of 200 cm/h, and a solution containing IgG which was eluted from the column was collected and neutralized. The recovery rate of IgG was 90%, which meets the standard for the commercial use for antibody purification and enables the reduction of the adsorbent amount to about a half of the amount necessary for a general operational condition.

### <Comparative Example 1>

In Comparative Example 1, a nucleophilic addition reaction to an aliphatic aldehyde in which the rate of the covalent bond formation was high and the protein orientation was not controlled was investigated.

A support having amino groups on its surface was reacted with a buffer solution (pH 8.3) of glutaraldehyde (1 wt%) at 37°C for two hours, and thus aliphatic aldehyde sites were introduced onto a surface of the support. Then, by reacting with a buffer solution (pH 10) of NαNα-carboxymethyllysine (5 wt%) at 37°C, nitrilotriacetic acid sites were introduced to a part of the aliphatic aldehyde sites. The proportion of the introduced nitrilotriacetic acid sites was controlled by the reaction time. Then, by reacting the support obtained with a 0.1 M aqueous solution of nickel chloride, the nitrilotriacetic acid sites were converted into nickel complexes. By reacting the support thus obtained with a buffer solution (pH 7.4) of protein A (0.01 wt%) having a His-tag site at 37°C for eight hours, a support on which protein A was immobilized was obtained.

### <Comparative Example 2>

In Comparative Example 2, a carboxylic acid activated by carbodiimide with which the rate of the covalent bond formation was high and the protein orientation was not controlled was investigated.

A support having carboxyl groups on its surface was reacted with a buffer solution (pH 5.8) of 1-ethyl-3-(3-dimethylaminopropyl)-carbodiimide hydrochloride (1 wt%), and thus the carboxyl groups on a surface of the support were activated. Then, by reacting with a buffer solution (pH 10) of NαNα-carboxymethyllysine (5 wt%) at 37°C, nitrilotriacetic acid sites were introduced to a part of the carboxyl groups activated. The proportion of the introduced nitrilotriacetic acid sites was controlled by the reaction time. Then, by reacting the support obtained with a 0.1 M aqueous solution of nickel chloride, the nitrilotriacetic acid sites were converted into nickel complexes. By reacting the support thus obtained with a buffer solution (pH7.4) of protein A (0.01 wt%) having a His-tag site at 37°C for eight hours, a support on which protein A was immobilized was obtained.

### <Comparative Example 3>

In Comparative Example 3, a control containing no orientation-controlling site was investigated.

A support having amino groups on its surface was reacted with an aqueous solution containing 1,4-butanediol diglycidyl ether (10 wt%) and potassium sulfate (0.4 M) at 60°C for eight hours, and thus epoxide sites were introduced onto a surface of the support. By reacting the support obtained with a buffer solution (pH 7.4) of protein A (0.01 wt%) having a His-tag site at 37°C for eight hours, a support on which protein A was immobilized was obtained.

### <Comparative Example 4>

In Comparative Example 4, a control containing no immobilization site was investigated.

A support having amino groups on its surface was reacted with an aqueous solution containing 1,4-butanediol diglycidyl ether (10 wt%) and potassium sulfate (0.4 M) at 60°C for eight hours, and thus epoxide sites were introduced onto a surface of the support. Then, by reacting with a buffer solution (pH 10) of NαNα-carboxymethyllysine (5 wt%) at 60°C for 16 hours, nitrilotriacetic acid sites were introduced to the epoxide sites. Then, by reacting the support obtained with a 0.1 M aqueous solution of nickel chloride, the nitrilotriacetic acid sites were converted into nickel complexes. By reacting the support thus obtained with a buffer solution (pH 7.4) of protein A (0.01 wt%) having a His-tag site at 37°C for eight hours, a support on which protein A was immobilized was obtained.

### <Comparative Example 5>

In Comparative Example 5, an adsorbent in which the protein orientation was not controlled was investigated.

An empty column with a diameter of 60 cm was filled with the adsorbent obtained according to Comparative Example 3 to a height of 20 cm with a PBS buffer solution (pH 7.4). When a buffer solution (100 L) containing IgG (1000 g) and contaminants was added to the purification column thus produced at a flow rate of 200 cm/h and the adsorbent was washed with a PBS buffer solution (pH 7.4) at a flow rate of 200 cm/h, IgG was not adsorbed to the purification column and a part of IgG leaked.

### <Comparison of Activities>

The antibody adsorption capacities of the supports obtained in Comparative Examples 1, 2, 3 and 4 and Examples 1, 2 and 3 are shown in Table 1.

**[Table 1]**

| Condition | Rate of covalent bond formation¹⁾ | Antibody adsorption capacity |
|---|---|---|
| Comparative Example 1 | 10⁵ [M⁻¹s⁻¹] | 16 [mg/mL] |
| Comparative Example 2 | 10⁴ [M⁻¹s⁻¹] | 18 [mg/mL] |
| Comparative Example 3²⁾ | 10³ [M⁻¹s⁻¹] | 20 [mg/mL] |
| Comparative Example 4³⁾ | - | <5 [mg/mL] |
| Example 1 | 10³ [M⁻¹s⁻¹] ^{4, 5)} | 25 [mg/mL] |
| Example 2 | 10³ [M⁻¹s⁻¹] ^{6, 7)} | 34 [mg/mL] |
| Example 3 | 10³ [M⁻¹s⁻¹] ^{5, 6)} | 38 [mg/mL] |

| | | |
|---|---|---|
| 1) The reaction rate constant was used as the index of the rate. 2) Without the orientation-controlling sites. 3) Without the immobilization sites. 4) The spacer lengths were 6 Å. 5) The proportion of the introduced orientation-controlling sites was about 40%. 6) The spacer lengths were 20 Å. 7) The proportion of the introduced orientation-controlling sites was about 23%. | | |

In the systems with large reaction rate constants of the covalent bond formation (Comparative Examples 1 and 2), the reversible bond formation is not likely to progress earlier and the effect of controlling the orientation is not sufficient. Thus, the antibody adsorption capacities are almost the same as that of the system without the orientation-controlling sites (Comparative Example 3). In addition, in the system without the immobilization sites (Comparative Example 4), because the protein is not immobilized, the antibody adsorption capacity is small. On the other hand, in the systems with small reaction rate constants of the covalent bond formation (Examples 1, 2 and 3), the reversible bond formation progresses earlier and the effect of controlling the orientation can be obtained. Thus, the antibody adsorption capacities are larger than those of Comparative Examples. In this regard, the effect of controlling the orientation varies with the spacer lengths and the proportion of the introduced orientation-controlling sites (Examples 1, 2 and 3). In Examples 1, 2 and 3, rates of the reversible bond formation are about 10⁴ [M⁻¹ s⁻¹], and the rates of the covalent bond formation are about 10³ [M⁻¹ s⁻¹]. A relation between the rates of the reversible bond formation and the rates of the covalent bond formation is influenced by the number of the covalent binding site, a reactivity of the immobilization site, pH of the solution, type of the orientation-controlling site, and type of the immobilization site. Among them, the type of the immobilization site gives the most influence. In addition, the reversible bond formation and the covalent bond formation may occur at the same time, or may not occur at the same time.

As it is obvious from the above embodiments, when the reversible binding site reversibly binds to the orientation-controlling site, the protein orientation is aligned, the reversible binding site being at a distance corresponding to the excluded volume of the target substance or longer from the adsorption site. By immobilizing the protein in this state by covalent binding, the orientation of the adsorption site is fixed. As a result, the utilization efficiency of the adsorption site of the protein improves, and the protein amount can be reduced and the step for storing the culture solution can be simplified due to the faster purification step.

## Claims

1. An adsorbent comprising:
a support (20); and
a protein (10)having an adsorption site(30) for adsorbing a target substance,
wherein the protein includes a reversible binding site(40) and a covalent binding site(50),
the support (20)includes an orientation-controlling site (70) forming a reversible bond with the reversible binding site(40), and an immobilization site (80) forming a covalent bond with the covalent binding site(50),
the covalent binding site(50) includes a nucleophilic functional group, and
the immobilization site (80) includes a functional group capable of reacting with the nucleophilic functional group by a nucleophilic substitution or a nucleophilic addition.

2. The adsorbent according to claim 1,
wherein the reversible binding site (40) contains two or more histidine residues and
the orientation-controlling site (70) contains a ligand forming a metal complex with a metal ion.

3. The adsorbent according to claim 2,
wherein the reversible binding site (40) contains six or more histidine residues.

4. The adsorbent according to claim 2 or 3,
wherein the metal ion is selected from the group consisting of iron, cobalt, nickel, copper and zinc, and
the ligand is selected from the group consisting of nitrilotriacetic acid, iminodiacetic acid and 1,4,7-triazacyclononane.

5. The adsorbent according to any of claims 1 to 4,
wherein the covalent binding site(50) is an alcohol, phenol, an amine, an imine, imidazole, a thiol, a sulfide, a disulfide, a carboxylic acid, or a conjugate base thereof.

6. The adsorbent according to any of claims 1 to 5,
wherein the support (20) includes a support base material (60),
the support base material(60) and the orientation-controlling site (70) are bound via a first spacer (90) having a length of 5 Å or more and the support base material (60) and the immobilization site (80)are bound via a second spacer (100)having a length of 5 Å or more.

7. The adsorbent according to claim 6,
wherein each of the first spacer (90)and the second spacer (100) contains an oligo ethylene glycol.

8. The adsorbent according to any of claims 1 to 7,
wherein the support base material is a polysaccharide, a synthetic resin or an inorganic compound.

9. The adsorbent according to claim 8,
wherein the polysaccharide is agarose, sepharose, cellulose or a derivative thereof,
the synthetic resin is a copolymer containing a polystyrene, a polyalkyl methacrylate, a polyglycidyl methacrylate, a polyvinyl alcohol, a polyvinylpyrrolidone, a polyacrylamide, a polysiloxane, a polyfluoroethylene, or a derivative thereof, and
the inorganic compound is an oxide, or a salt or a hydrogen salt of carbonic acid, phosphoric acid, boric acid, silicic acid or sulfuric acid of silicon, aluminum, titanium, iron, cobalt, zinc or zirconium.

10. The adsorbent according to claim 8,
wherein the inorganic compound is silica, alumina, titania, zirconia, iron oxide, silicate, ferrite or hydroxyapatite.
